# EUROPEAN PATENT SPECIFICATION

(11) **EP 1 234 576 B1**
(45) Date of publication and mention of the grant of the patent: **08.03.2006**
(21) Application number: 00970169.9
(22) Date of filing: 27.10.2000
(51) Int. Cl.: A61K 31/522, A61P 1/14

(54) **PHARMACEUTICAL COMPOSITION FOR EATING DISORDERS**
ARZNEIMITTEL GEGEN ESSSTÖRUNGEN
REMEDES CONTRE LES TROUBLES DE L'ALIMENTATION

(30) Priority: 29.10.1999 JP 31013899
(43) Date of publication of application: 28.08.2002
(73) Proprietor: KYOWA HAKKO KOGYO CO., LTD., Chiyoda-ku, Tokyo 100-8185 (JP)
(72) Inventor: HARA, Takuji, Kyowa Hakko Kogyo Co., Ltd, Ube-shi, Yamaguchi 755-8501 (JP); ISHIKAWA, Yumiko Kyowa Hakko Kogyo Co. Ltd., Shizuoka 411-8731 (JP); RYOMOTO, Tetsuya, Kyowa Hakko Kogyo Co., Ltd, Ube-shi, Yamaguchi 755-8501 (JP)
(74) Representative: Vossius & Partner
(86) International application number: PCT/JP2000/007586
(87) International publication number: WO 2001/032182

(56) References cited:
- EP-A- 0 559 893
- EP-A- 0 590 919
- EP-A- 0 607 607
- EP-A1- 0 590 919
- EP-A1- 0 607 607
- WO-A1-95/33750
- US-A- 2 729 642
- US-A- 4 593 095

## Description

The present invention relates to therapeutic agents for eating disorders.

Most of the compounds represented by formula (I) shown below and compounds related thereto are known compounds, and their adenosine A₂-receptor antagonism, anti-Parkinson's disease action, anti-depressive action, anti-asthmatic action, inhibitory action on bone absorption, action on central excitation and inhibitory action on neurodegeneration are known [JP 47-26516 B, J. Med. Chem., 34, 1431 (1991), J. Med. Chem., 36, 1333 (1993); WO 92/06976, JP 6-211856 A, JP 6-239862 A, WO 95/23165, JP 6-16559 A, WO 94/01114 and WO 99/12546].

EP-A-0 590 919 discloses therapeutic agents comprising a xanthine derivative to be used for treating Parkinson's disease.

EP-A-0 559 893 and EP-A-0 607 607 relate to xanthine derivatives having adenosine A₂ receptor-antagonistic activity.

US-A-4 593 095 relates to 8-arylxanthine derivatives having adenosine receptor-antagonistic activity.

US-A-2 729 642 relates to a water soluble acid salt of 8-(para-aminobenzyl)caffeine useful as hypotensive agent.

However, it is not known that said compounds have aperitive activity.

An object of the present invention is to provide excellent therapeutic agents for eating disorders.

The present invention relates to those described below.
(1) Use of a xanthine derivative represented by formula (I): 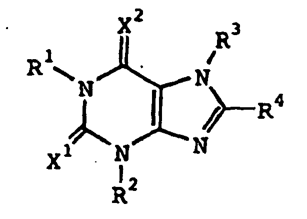 wherein R¹, R² and R³ independently represent hydrogen, lower alkyl, lower alkenyl or lower alkynyl; R⁴ represents cycloalkyl, -(CH₂)ₙ-R⁵ (wherein R⁵ represents substituted or unsubstituted aryl, or a substituted or unsubstituted heterocyclic group, and n is an integer of 0 to 4), or the following group: 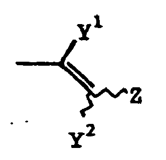
   wherein Y¹ and Y² independently represent hydrogen, halogen or lower alkyl, and Z represents substituted or unsubstituted aryl, the following group: 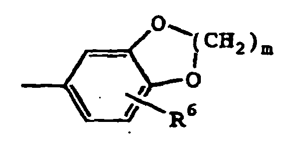
   (wherein R⁶ represents hydrogen, hydroxy, lower alkyl, lower alkoxy, halogen, nitro or amino, and m is an integer of 1 to 3), or a substituted or unsubstituted heterocyclic group; and X¹ and X² independently represent O or S, or a pharmaceutically acceptable salt thereof for the production of an agent for treating or preventing eating disorders.
(2) The use of the xanthine derivative according to the above (1) or a pharmaceutically acceptable salt thereof wherein X¹ and X² are O.
(3) The use of the xanthine derivative according to the above (1) or (2) or a pharmaceutically acceptable salt thereof wherein R⁴ is the following group: 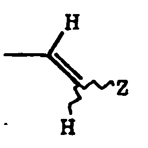
   wherein Z has the same meaning as defined above.
(4) Use of the xanthine derivative according to any one of the above (1) to (3) or a pharmaceutically acceptable salt thereof for the production of an aperitive.
(5) Use of the xanthine derivative according to any one of the above (1) to (3) or a pharmaceutically acceptable salt thereof for the production of an agent for treating or preventing anorexia.

Hereinafter, the compound represented by formula (I) is referred to as compound (I).

In the definition of compound (I), the lower alkyl and the lower alkyl moiety in the lower alkoxy mean a straight-chain or branched C₁-C₆ alkyl group such as methyl, ethyl, propyl, isopropyl, butyl, isobutyl, sec-butyl, tert-butyl, pentyl, neopentyl or hexyl; the lower alkenyl means a straight-chain or branched C₂-C₆ alkenyl group such as vinyl, allyl, methacryl, crotyl, 3-butenyl, 2-pentenyl, 4-pentenyl, 2-hexenyl or 5-hexenyl; the lower alkynyl means a straight-chain or branched C₂-C₆ alkynyl group such as ethynyl, propargyl, 2-butynyl, 3-butynyl, 2-pentynyl, 4-pentynyl, 2-hexynyl, 5-hexynyl or 4-methyl-2-pentynyl; the aryl means phenyl or naphthyl; the cycloalkyl means a C₃-C₈ cycloalkyl group such as cyclopropyl, cyclobutyl, cyclopentyl, cyclohexyl, cycloheptyl or cyclooctyl; examples of the heterocyclic group are furyl, thienyl, pyrrolyl, pyranyl, thiopyranyl, pyridyl, thiazolyl, imidazolyl, pyrimidinyl, triazinyl, indolyl, quinolyl, purinyl and benzothiazolyl; and the halogen includes fluorine, chlorine, bromine and iodine. The substituted aryl and the substituted heterocyclic group have 1 to 3 independently-selected substituents such as lower alkyl, hydroxy, substituted or unsubstituted lower alkoxy, halogen, nitro, amino, lower alkylamino, di(lower alkyl)amino, trifluoromethyl, trifluoromethoxy, aralkyl, aralkyloxy, aryl, aryloxy, lower alkanoyl, lower alkanoyloxy, aroyl, arayloxy, arylalkanoyloxy, carboxy, lower alkoxycarbonyl, lower alkylcarbamoyl, di(lower alkyl)carbamoyl, sulfo, lower alkoxysulfonyl, lower alkylsulfamoyl or di(lower alkyl)sulfamoyl. The lower alkyl and the alkyl moiety in the lower alkoxy, lower alkyl amino, di(lower alkyl)amino, lower alkanoyl, lower alkanoyloxy, lower alkoxycarbonyl, lower alkylcarbamoyl, di(lower alkyl)carbamoyl, lower alkoxysulfonyl, lower alkylsulfamoyl and di(lower alkyl)sulfamoyl have the same meaning as the lower alkyl defined above. The halogen has the same meaning as defined above. The aryl and the aryl moiety in the aryloxy have the same meaning as the aryl defined above. Examples of the aralkyl and the aralkylmoiety in the aralkyloxy are benzyl and phenethyl. Examples of the aroyl and the aroyl moiety in the aroyloxy are benzoyl and naphthoyl. Examples of the arylalkyl moiety in the arylalkanoyloxy are benzyl and phenethyl. Examples of the substituents for the substituted lower alkoxy are hydroxy, lower alkoxy, halogen, amino, azido, carboxy and lower alkoxycarbonyl. The alkyl moiety in the lower alkoxy and lower alkoxycarbonyl has the same meaning as the lower alkyl defined above, and the halogen has the same meaning as the halogen defined above.

The pharmaceutically acceptable salts of compound (I) include pharmaceutically acceptable acid addition salts, metal salts, ammonium salts, organic amine addition salts and amino acid addition salts.

The pharmaceutically acceptable acid addition salts of compound (I) include inorganic acid addition salts such as hydrochloride, sulfate and phosphate, and organic acid addition salts such as acetate, maleate, fumarate, tartrate, citrate and methanesulfonate; the pharmaceutically acceptable metal salts include alkalimetal salts such as sodium salt and potassium salt, alkaline earth metal salts such as magnesium salt and calcium salt, aluminum salt, and zinc salt. The pharmaceutically acceptable ammonium salts include ammonium and tetramethylammonium. The pharmaceutically acceptable organic amine addition salts include a salt with morpholine or piperidine; and the pharmaceutically acceptable amino acid addition salts include a salt with lysine, glycine or phenylalanine.

Compound (I) including novel compounds can be produced by the methods disclosed in the above-mentioned publications or according to other methods. The desired compound in the process can be isolated and purified by purification methods conventionally used in synthetic organic chemistry, such as filtration, extraction, washing, drying, concentration, recrystallization or various kinds of chromatography.

In the case where a salt of compound (I) is desired and it is produced in the form of a desired salt, it may be subjected to purification as such. In the case where compound (I) is produced in the free form and its salt is desired, it is dissolved or suspended in a suitable solvent, and then an acid or a base may be added thereto to form the salt.

Compound (I) and pharmaceutically acceptable salts thereof may be in the form of an adduct with water or various solvents, which can also be used as the therapeutic agent of the present invention.

Some of compounds (I) have optical isomers, and all potential stereoisomers and mixtures thereof can also be used as the therapeutic agent of the present invention.

Examples of compound (I) are shown in Table 1.

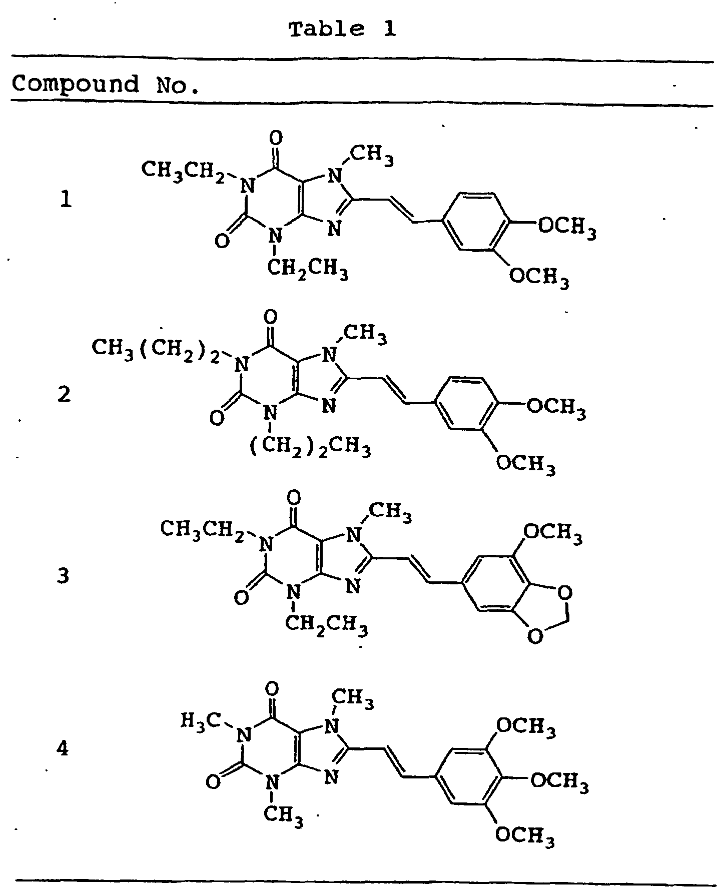

Compound 1: (E)-8-(3,4-dimethoxystyryl)-1,3-diethyl-7-methylxanthine (JP 6-211856 A)
Melting point: 190.4-191.3 °C

**Elemental analysis: C₂₀H₂₄N₄O₄**

| | | | |
|---|---|---|---|
| Calcd (%): | C 62.48, | H 6.29, | N 14.57 |
| Found (%): | C 62.52, | H 6.53, | N 14.56 |

IR( KBr) νmax(cm⁻¹): 1697, 1655, 1518
NMR(CDCl₃, 270MHz) δ(ppm): 7.74(1H, d, J=15.5Hz), 7.18(1H, dd, J=8.3, 1.9Hz), 7.08(1H, d, J=1.9Hz), 6.89(1H, d, J=8.3Hz), 6.77(1H, d, J=15.5Hz), 4.21(2H, q, J=6.9Hz), 4.09(2H, q, J=6.9Hz), 4.06(3H, s), 3.96(3H, s), 3.93(3H, s), 1.39(3H, t, J=6.9Hz), 1.27(3H, t, J=6.9Hz)
Compound 2: (E)-8-(3,4-dimethoxystyryl)-7-methyl-1,3-dipropylxanthine (WO 92/06976)
Melting point: 164.8-166.2 °C (Recrystallization from 2-propanol/water)

**Elemental analysis: C₂₂H₂₈N₄O₄**

| | | | |
|---|---|---|---|
| Calcd. (%): | C 64.06, | H 6.84, | N 13.58 |
| Found (%): | C 64.06, | H 6.82, | N 13.80 |

IR(KBr) νmax(cm⁻¹): 1692, 1657
NMR(DMSO-d₆, 270MHz) δ(ppm): 7.60(1H, d, J=15.8Hz), 7.40(1H, d, J=2.0Hz), 7.28 (1H, dd, J=2.0, 8.4Hz), 7.18(1H, d, J=15.8Hz), 6.99(1H, d, J=8.4Hz), 4.02(3H, s), 3.99(2H, t), 3.90-3.80(2H, m), 3.85(3H, s), 3.80(3H, s), 1.85-1.50(4H, m), 1.00-0.85(6H, m)
Compound 3: (E)-1,3-diethyl-8-(3-methoxy-4,5-methylenedioxy styryl)-7-methylxanthine (JP 6-211856 A)
Melting point: 201.5-202.3 °C

**Elemental analysis: C₂₀H₂₂N₄O₅**

| | | | |
|---|---|---|---|
| Calcd. (%): | C 60.29, | H 5.57, | N 14.06 |
| Found (%): | C 60.18, | H 5.72, | N 13.98 |

IR(KBr) νmax(cm⁻¹): 1694, 1650, 1543, 1512, 1433
NMR(DMSO-d₆, 270MHz) δ(ppm): 7.58(1H, d, J=15.8Hz), 7.23(1H, d, J=15.8Hz), 7.20(1H, d, J=1.0Hz), 7.09(1H, d, J=1.0Hz), 6.05(2H, s), 4.09-4.02(2H, m), 4.02(3H, s), 3.94-3.89(2H, m), 3.89(3H, s), 1.25(3H, t, J=7.2Hz), 1.13(3H, t, J=6.9Hz)
Compound 4: (E)-8-(3,4,5-trimethoxystyryl)caffeine (JP 47-26516 B)
IR(KBr) νmax(cm⁻¹): 1702, 1667, 1508, 1432
NMR(DMSO-d₆, 270MHz) δ(ppm): 7.61(1H, d, J=16.0Hz), 7.25(1H, d, J=16.0Hz), 7.09(2H, s), 4.03(3H, s), 3.85(6H, s), 3.71(3H, s), 3.45(3H, s), 3.21(3H, s)
MS(EI) 386(M⁺)

Hereinafter, the pharmacological activity of compound (I) is shown by the following Test Examples.

### Test Example 1: Action of increasing feed intake by 4-week oral administration.

### 1. Preparation of a solution containing a test compound

Compound 1 was suspended in a 0.5 % aqueous methyl cellulose solution to a final concentration of 0.6 mg/mL for 6 mg/kg dose, 3 mg/mL for 30 mg/kg dose, 16 mg/mL for 160 mg/kg dose, or 80 mg/mL for 800 mg/kg dose, and the resulting solution was used.

### 2. Animals used

Male and female Crj:CD (SD) rats (SPF) at the age of 5 weeks were purchased from Nippon Charles River Co., Ltd., and were then fed and conditioned for 9 days. During the term, their body weight change and conditions were observed, and animals determined as in healthy conditions were used at the test. At the start of the use, the body weights of the male animals were within a range of 187 to 209 g, while those of the female animals were within a range of 147 to 173 g. During both of the conditioning and feeding term and the test term, the animals were fed with a solid feed for mouse and rat (CRF-1 15 kGy, Oriental Yeast Industry, Co., Ltd.) and with water ad libitum.

### 3. Composition of test groups

As to the number of the animals used, 15 each of male and female animals were used for each group. As a negative control group, a group administered a 0.5 % aqueous methyl cellulose solution alone was prepared. Based on the body weight at the termination of the conditioning and feeding term, the animals were randomly assigned to individual groups, so that the body weights were uniformly distributed among the resulting individual groups.

### 4. Administration method and administration term

Oral administration was selected as the administration route. The solvent or a test compound-containing solution was administered once daily in the morning for 4 weeks in a dose of 1 mL per 100 g of body weight.

### 5. Test results

The feed intake of the test animals was measured on days 7, 14, 21 and 28. The measured values were tested for homoscedasticity by the Bartlett's test. When the variance was homogeneous, one-way layout analysis of variance was performed; in the case that significance was observed, herein, the control group and the individual dosed groups were tested by the Dunnett's test. When the variance was not homogeneous, the Kruskal-Wallis's rank test was performed; in the case that significance was observed among the groups, the Dunnett's test was conducted.

The results are shown in Table 2.

**Table 2**

| Compound | Dose (mg/kg, po) | Sex | Feed intake (g ±: SD) | |
|---|---|---|---|---|
| | | | day 7 | day 14 |
| Solvent control | - | male | 23.50±1.53 | 24.62±1.96 |
| (0.5 % methyl cellulose-dosed group) | | | | |
| 1 | 6 | male | 21.45±1.28 | 23.97±1.21 |
| 1 | 30 | male | 23.54±2.15 | 26.12±2.03 |
| 1 | 160 | male | 23.94±1.58 | 26.20±1.77 |
| 1 | 800 | male | 22.84±1.81 | 25.40±2.42 |
| Solvent control | - | female | 16.66±1.29 | 18.09±1.54 |
| (0.5 % methyl cellulose-dosed group) | | | | |
| 1 | 6 | female | 15.93±1.09 | 18.03±1.37 |
| 1 | 30 | female | 17.24±1.19 | 19.38±1.74 |
| 1 | 160 | female | 17.73±1.40 | 19.56±1.68* |
| 1 | 800 | female | 17.30±0.99 | 19.19±1.44 |
| | | | | |
| | | | | |

| Compound | Dose (mg/kg, po) | Sex | Feed intake (g ± SD) | |
|---|---|---|---|---|
| | | | day 21 | day 28 |
| Solvent control | - | male | 25.39±1.91 | 25.12±2.09 |
| (0.5 % methyl cellulose-dosed group) | | | | |
| 1 | 6 | male | 24.96±1.58 | 25.23±1.80 |
| 1 | 30 | male | 27.03±2.07 | 27.37±2.56* |
| 1 | 160 | male | 27.31±1.74* | 27.81±2.47** |
| 1 | 800 | male | 26.67±2.19 | 27.03±2.37 |
| Solvent control | - | female | 18.47±1.91 | 18.14±1.56 |
| (0.5 % methyl cellulose-dosed group) | | | | |
| 1 | 6 | female | 18.76±1.18 | 18.80±1.46 |
| 1 | 30 | female | 19.93±2.08 | 20.50±1.96** |
| 1 | 160 | female | 20.56±1.73* | 20.87±2.04** |
| 1 | 800 | female | 20.43±1.69* | 21.13±1.76** |

| | | | | |
|---|---|---|---|---|
| * : P ≦ 0.05 (compared with the control group) | | | | |
| ** :P ≦ 0.01 (compared with the control group) | | | | |

The test results show that the 4-week administration of Compound 1 increased the feed intake both in the males and in the females.

### Test Example 2: Action of increasing feed intake and body weight by 4-week oral administration

### 1. Preparation of a solution containing a test compound

Compound 1 was suspended in a 0.5 % aqueous methyl cellulose solution to a final concentration of 20 mg/mL for 200 mg/kg dose or 40 mg/mL for 400 mg/kg dose, and the resulting solution was used.

### 2. Animals used

Male and female Crj : CD (SD) rats (SPF) at the age 5 of weeks were purchased from Nippon Charles River Co., Ltd., and were then fed and conditioned for 7 days. During the term, their body weight change and conditions were observed, and animals determined as in healthy conditions were used at the test. At the start of the use, the body weights of the male animals were within a range of 177.4 to 193.6 g, while those of the female animals were within a range of 141.1 to 160.3 g. During both of the conditioning and feeding term and the test term, the animals were fed with a solid feed for mouse and rat [FR-2, Funabashi Agricultural Farm Co., Ltd.] and with water ad libitum.

### 3. Composition of test groups

As to the number of the animals used, 5 each of male and female animals were used for each group. As a negative control group, a group administered a 0.5 % aqueous methyl cellulose solution alone was prepared. Based on the body weight at the termination of the conditioning and feeding term, the animals were randomly assigned to individual groups, so that the body weights were uniformly distributed among the resulting individual groups.

### 4. Administration method and administration term

oral administration was selected as the administration route. The solvent or the test compound was administered once daily in the morning for 4 weeks in a dose of 1 mL per 1.00 g of body weight.

### 5. Test results

The body weights and feed intake of the test animals were measured on days 0, 7, 14, 21 and 28. The individual measured values were tested, based on the same standards as in the Test Example 1.

The results are shown in Table 3 (body weight change) and Table 4 (feed intake change).

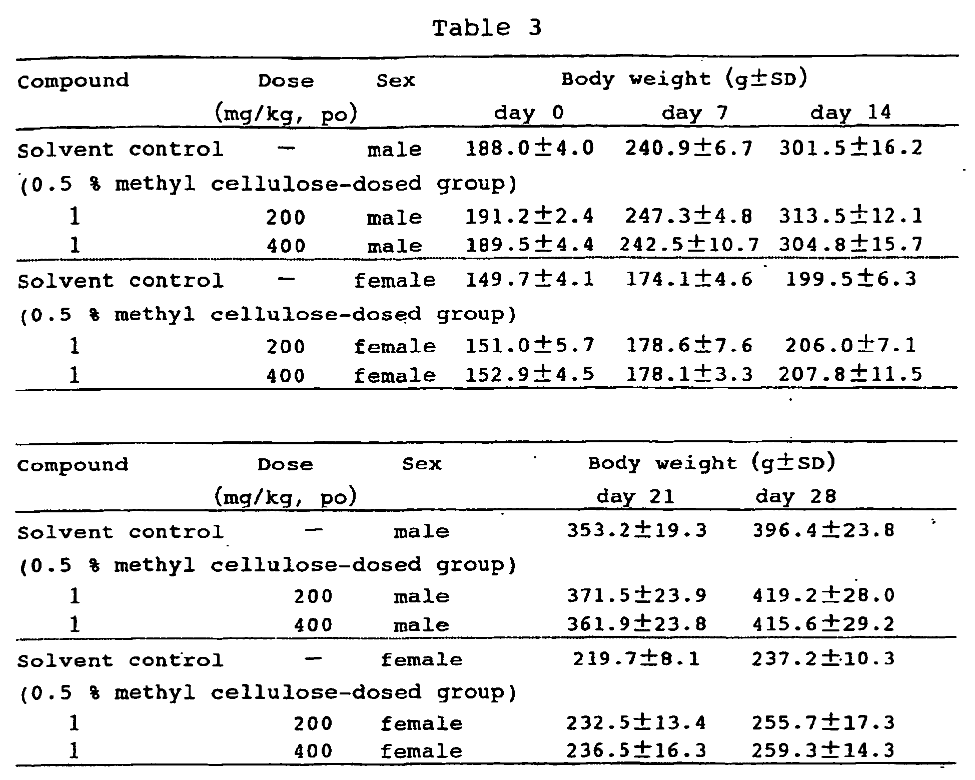

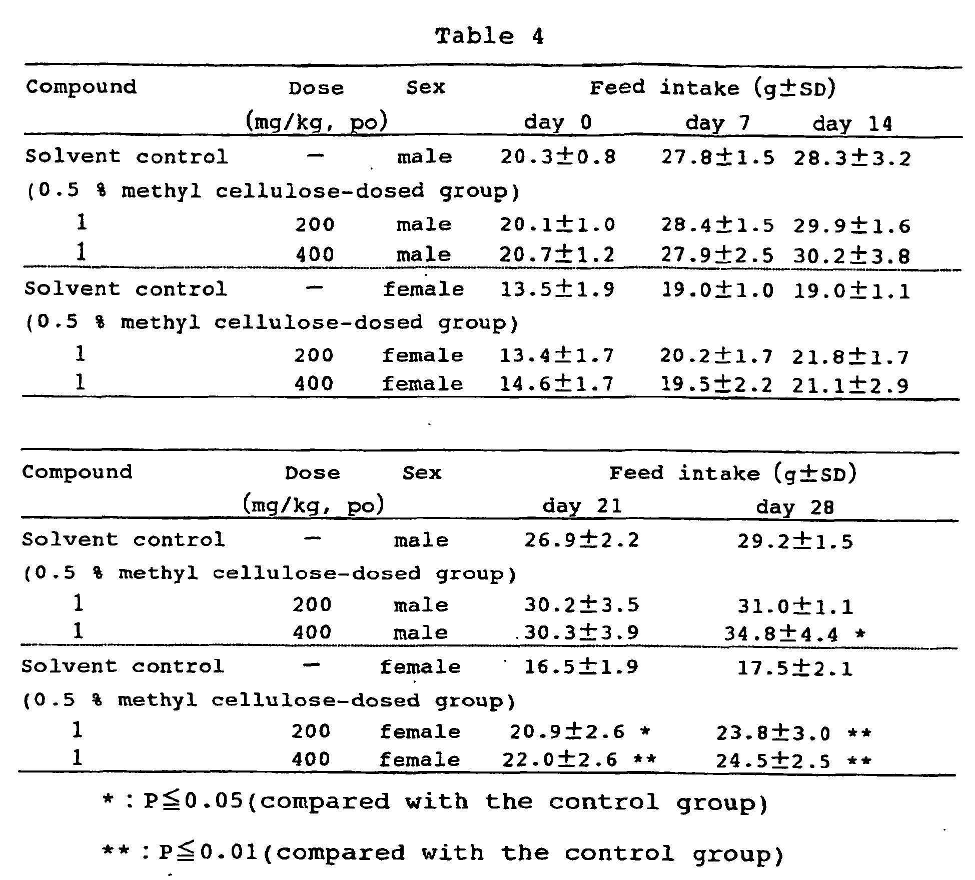

The test results show that the 4-week administration of Compound 1 increased the feed intake and body weight both in the males and in the females.

### Test Example 3: Acute toxicity test

Test compounds were orally administered to groups of dd-strain male mice weighing 20 ± 1 g, each group consisting of three mice. Seven days after the administration, the mortality was observed to determine a minimum lethal dose (MLD) of each compound.

The MLD value of Compound 1 was greater than 1000 mg/kg.

Compound (I) or pharmaceutically acceptable salts thereof have an action of increasing feed intake and body weight. Thus, compound (I) or pharmaceutically acceptable salts are useful as a therapeutic agent for eating disorders, such as anorexia nervosa (cibophobia, absolute anorexia nervosa).

Compound (I) or pharmaceutically acceptable salts thereof can be used as such or in the form of various pharmaceutical compositions. The pharmaceutical compositions used in the present invention can be prepared by uniformly mixing an effective amount of compound (I) or a pharmaceutically acceptable salt thereof as an active ingredient with pharmaceutically acceptable carriers. The pharmaceutical compositions are preferably in a unit dosage form suitable for rectal administration, oral or parenteral (including subcutaneous, intravenous and intramuscular administration) administration, etc.

For preparing a pharmaceutical composition for oral administration, any useful pharmaceutically acceptable carriers can be used. For example, liquid preparations for oral administration such as suspension and syrup can be prepared using water; sugars such as sucrose, sorbitol or fructose; glycols such as polyethylene glycol or propylene glycol; oils such as sesame oil, olive oil or soybean oil; preservatives such as a p-hydroxybenzoate; flavors such as strawberry flavor or peppermint, etc. Powder, pills, capsules and tablets can be prepared using excipients such as lactose, glucose, sucrose or mannitol; disintegrators such as starch or sodium alginate; lubricants such as magnesium stearate or talc; binders such as polyvinyl alcohol, hydroxypropyl cellulose or gelatin; surfactants such as fatty acid esters; plasticizers such as glycerin, etc. Tablets and capsules are the most useful oral unit dosage because of the readiness of administration. For preparing tablets and capsules, solid pharmaceutical carriers are used.

Injections can be prepared using carriers such as distilled water, a salt solution, a glucose solution or a mixture of a salt solution and a glucose solution. The preparation can be prepared in the form of a solution, suspension or dispersion by using a suitable auxiliary according to a conventional method.

Compound (I) or a pharmaceutically acceptable salt thereof can be administered orally in the pharmaceutical composition described above or parenterally as the injection or the like. The effective dose and administration schedule vary depending on the mode of administration, the age, the weight of a patient, symptoms of the disease, etc. However, generally, compound (I) or a pharmaceutically acceptable salt thereof is administered in a dose of 1 to 900 mg/60 kg/day, preferably in a dose of 1 to 200 mg/60 kg/day, at one time or in several parts.

Certain embodiments of the present invention are described in the following examples.

### Example 1: Tablets

Tablets having the following composition are prepared in a conventional manner.

Compound 1 (40 g) is mixed with 286.8 g of lactose and 60 g of potato starch, followed by addition of 120 g of a 10% aqueous solution of hydroxypropyl cellulose. The resultant mixture is kneaded, granulated, and then dried by a conventional method. The granules are refined to give granules used to make tablets. After mixing the granules with 1.2 g of magnesium stearate, the mixture is formed into tablets each containing 20 mg of the active ingredient by using a tablet maker (Model RT-15, Kikusui) having pestles of 8 mm diameter.

**Composition**

| | |
|---|---|
| Compound 1 | 20 mg |
| Lactose | 143.4 mg |
| Potato Starch | 30 mg |
| Hydroxypropyl Cellulose | 6 mg |
| Magnesium Stearate | 0.6 mg |
| | 200 mg |

### Example 2: Capsules

Capsules having the following composition are prepared in a conventional manner.

Compound 1 (200 g) is mixed with 995 g of Avicel and 5 g of magnesium stearate. The mixture is put in hard capsules No. 4 each having a capacity of 120 mg by using a capsule filler (Model LZ-64, Zanashi) to give capsules each containing 20 mg of the active ingredient.

**Composition**

| | |
|---|---|
| Compound 1 | 20 mg |
| Avicel | 99.5 mg |
| Magnesium Stearate | 0.5 mg |
| | 120 mg |

### Example 3: Injections

Injections having the following composition are prepared in a conventional manner.

Compound 1 (1 g) is dissolved in 100 g of purified soybean oil, followed by addition of 12 g of purified egg yolk lecithin and 25 g of glycerin for injection. The resultant mixture is made up to 1,000 ml with distilled water for injection, thoroughly mixed, and emulsified by a conventional method. The resultant dispersion is subjected to aseptic filtration by using 0.2 µm disposable membrane filters, and then aseptically put into glass vials in 2 ml portions to give injections containing 2 mg of the active ingredient per vial.

**Composition**

| | |
|---|---|
| Compound 1 | 2 mg |
| Purified Soybean Oil | 200 mg |
| Purified Egg Yolk Lecithin | 24 mg |
| Glycerine for Injection | 50 mg |
| Distilled Water for Injection | 1.72 ml |
| | 2.00 ml |

### Example 4: Anal suppository

Formulations for rectal administration having the following composition are prepared in a conventional manner.

Witepsol® H15 (678.8 g manufactured by Dynamit Nobel, Ltd.) and Witepsol® E75 (290.9 g, manufactured by Dynamit Nobel, Ltd.) are melted at 40 to 50 °C. In the resulting molten mixture are uniformly mixed and dispersed Compound I (2.5 g), potassium dihydrogen phosphate (13.6 g) and disodium hydrogen phosphate (14.2 g). The resulting dispersion is poured into plastic suppository molds, and gradually cooled to give anal suppositories containing 2.5 mg of the active ingredient per formulation.

**Composition**

| | |
|---|---|
| Compound 1 | 2.5 mg |
| Witepsol® H15 | 678.8 mg |
| Witepsol® E75 | 290.9 mg |
| Potassium dihydrogen phosphate | 13.6 mg |
| Disodium hydrogen phosphate | 14.2 mg |
| | 1000 mg |

The present invention provides therapeutic agents for eating disorders, comprising a xanthine derivative or a pharmaceutically acceptable salt thereof as an active ingredient.

## Claims

1. Use of a xanthine derivative represented by formula (I): 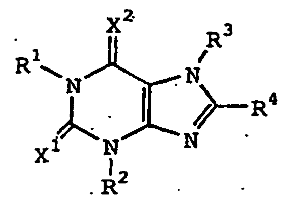
wherein R¹, R² and R³ independently represent hydrogen, lower alkyl, lower alkenyl or lower alkynyl; R⁴ represents cycloalkyl;. -(CH₂)ₙ-R⁵ (wherein R⁵ represents substituted or unsubstituted aryl, or a substituted or unsubstituted heterocyclic group, and n is an integer of 0 to 4), or the following group: 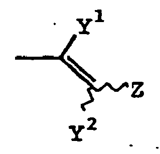
wherein Y¹ and Y² independently represent hydrogen, halogen or lower alkyl, and Z represents substituted or unsubstituted aryl, the following group: 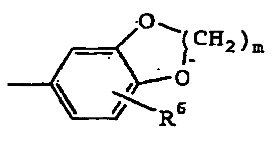
(wherein R⁶ represents hydrogen, hydroxy, lower alkyl, lower alkoxy, halogen, nitro or amino, and m is an integer of 1 to 3), or a substituted or unsubstituted heterocyclic group; and X¹ and X² independently represent O or S, or a pharmaceutically acceptable salt thereof for the production of an agent for treating or preventing eating disorders.

2. The use of the xanthine derivative according to claim 1 or a pharmaceutically acceptable salt thereof wherein X¹ and X² are O.

3. The use of the xanthine derivative according to claim 1 or 2 or a pharmaceutically acceptable salt thereof wherein R⁴ is the following group: 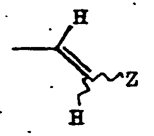
wherein Z has the same meaning as defined above.

4. Use of the xanthine derivative according to any one of claims 1 to 3 or a pharmaceutically acceptable salt thereof for the production of an aperitive agent.

5. Use of the xanthine derivative according to any one of claims 1 to 3 or a pharmaceutically acceptable salt thereof for the production of an agent for treating or preventing anorexia.

## Patentansprüche

1. Verwendung eines Xanthinderivats der Formel (I): 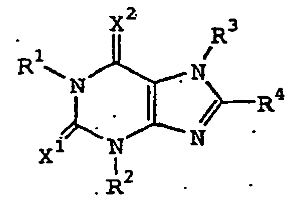
wobei R¹, R² und R³ unabhängig voneinander Wasserstoff, Niederalkyl, Niederalkenyl oder Niederalkinyl bedeuten; R⁴ Cycloalkyl, -(CH₂)ₙ-R⁵ (wobei R⁵ substituiertes oder unsubstituiertes Aryl oder einen substituierten oder unsubstituierten heterocyclischen Rest bedeutet, und n eine ganze Zahl von 0 bis 4 ist) oder den nachstehenden Rest: 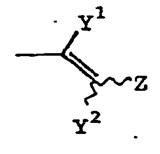
bedeutet, wobei Y¹ und Y² unabhängig voneinander Wasserstoff, Halogen oder Niederalkyl bedeuten, und Z substituiertes oder unsubstituiertes Aryl, den nachstehenden Rest: 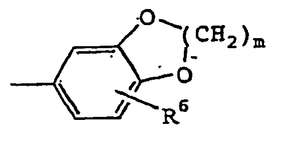
(wobei R⁶ Wasserstoff, Hydroxy, Niederalkyl, Niederalkoxy, Halogen, Nitro oder Amino bedeutet, und m eine ganze Zahl von 1 bis 3 ist) oder einen substituierten oder unsubstituierten heterocyclischen Rest bedeutet; und X¹ und X² unabhängig voneinander O oder S bedeuten, oder eines pharmazeutisch verträglichen Salzes davon zur Herstellung eines Mittels zur Behandelung oder Vorbeugung von Essstörungen.

2. Verwendung des Xanthinderivats nach Anspruch 1 oder eines pharmazeutisch verträglichen Salzes davon, wobei X¹ und X² O sind.

3. Verwendung des Xanthinderivats nach Anspruch 1 oder 2 oder eines pharmazeutisch verträglichen Salzes davon, wobei R⁴ der nachstehende Rest: 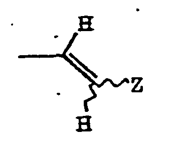
ist, wobei Z dieselbe Bedeutung wie vorstehend definiert hat.

4. Verwendung des Xanthinderivats nach einem der Ansprüche 1 bis 3 oder eines pharmazeutisch verträglichen Salzes davon zur Herstellung eines appetitanregenden Mittels.

5. Verwendung des Xanthinderivats nach einem der Ansprüche 1 bis 3 oder eines pharmazeutisch verträglichen Salzes davon zur Herstellung eines Mittels zur Behandelung oder Vorbeugung von Anorexie.

## Revendications

1. Utilisation d'un dérivé de xanthine représenté par la formule (I) : 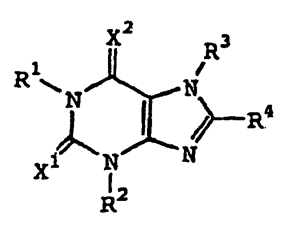
dans laquelle R¹, R² et R³ représentent indépendamment un hydrogène, un alkyle inférieur, un alkényle inférieur ou un alcynyle inférieur; R⁴ représente cycloalkyle, -(CH₂)ₙ-R⁵ (où R⁵ représente un aryle substitué ou non substitué ou un groupe hétérocyclique substitué ou non substitué, et n est un nombre entier de 0 à 4), ou le groupe suivant : 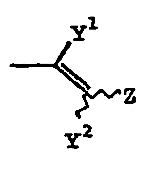
dans lequel Y¹ et Y² représentent indépendamment un hydrogène, un halogène ou un alkyle inférieur, et Z représente un aryle substitué ou non substitué, le groupe suivant : 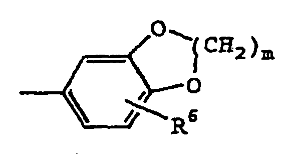
(dans lequel R⁶ représente un hydrogène, un hydroxy, un alkyle inférieur, un alkoxy inférieur, un halogène, un nitro ou un amino, et m est un nombre entier de 1 à 3), ou un groupe hétérocyclique substitué ou non substitué ; et X¹ et X² représentent indépendamment O ou S, ou un de ses sels pharmaceutiquement acceptable pour la production d'un agent pour traiter ou prévenir des troubles de l'alimentation.

2. Utilisation du dérivé de xanthine selon la revendication 1 ou d'un de ses sels pharmaceutiquement acceptables dans lequel X¹ et X² sont O.

3. Utilisation du dérivé de xanthine selon la revendication 1 ou la revendication 2 ou d'un de ses sels pharmaceutiquement acceptables dans lequel R⁴ est le groupe suivant : 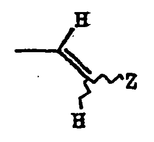
dans lequel Z a la même définition que ci-dessus.

4. Utilisation du dérivé de xanthine selon l'une quelconque des revendications 1 à 3 ou d'un de ses sels pharmaceutiquement acceptables pour la production d'un agent ayant un effet de stimulation de l'appétit.

5. Utilisation du dérivé de xanthine selon l'une quelconque des revendications 1 à 3 ou d'un de ses sels pharmaceutiquement acceptables pour la production d'un agent pour traiter ou prévenir l'anorexie.
